# EUROPEAN PATENT APPLICATION

(11) **EP 4 464 689 A1**
(43) Date of publication of application: **20.11.2024**
(21) Application number: 23903719.5
(22) Date of filing: 20.10.2023
(51) Int. Cl.: C07C 211/27, A01N 33/12, C07C 309/65

(54) **ANTIBACTERIAL COMPOSITION**

(30) Priority: 14.12.2022 KR 20220175181; 19.10.2023 KR 20230140150
(71) Applicant: LG Chem, Ltd., Yeongdeungpo-gu Seoul 07336 (KR)
(72) Inventor: LEE, Seungmo, Daejeon 34122 (KR); BAEK, Leehyeon, Daejeon 34122 (KR); LEE, Jeong Yun, Daejeon 34122 (KR); HUR, Yoon Hyung, Daejeon 34122 (KR); LEE, Jiyoung, Daejeon 34122 (KR); CHOI, Doowhan, Daejeon 34122 (KR)
(74) Representative: Plasseraud IP
(86) International application number: PCT/KR2023/016313
(87) International publication number: WO 2024/128515

(57) **Abstract**

The present invention relates to an antibacterial composition including a compound represented by Chemical Formula 1, and the antibacterial composition has improved thermal stability and antibacterial activity.

## Description

### [Technical Field]

The present specification relates to an antibacterial composition including a compound.

This application claims priority to and the benefit of Korean Patent Application Nos. 10-2022-0175181 and 10-2023-0140150 filed in the Korean Intellectual Property Office on December 14, 2022 and October 19, 2023, respectively, the entire contents of which are incorporated herein by reference.

### [Background Art]

Recently, various products such as daily supplies or hygiene products are required to have high antibacterial properties.

The degree of antibacterial properties required and the material requirements for imparting antibacterial properties differ depending on the material of the product requiring antibacterial properties and the state of final use. For example, the properties of the material for imparting antibacterial properties and the degree of antibacterial properties vary depending on the amount of antibacterial material applied to a product used and the materials used together.

Therefore, there is a need for developing an antibacterial material suitable for application to each of the various products.

### [Detailed Description of the Invention]

### [Technical Problem]

An exemplary embodiment of the present invention relates to an antibacterial composition, and more specifically, an object thereof is to provide an antibacterial composition including a quaternary ammonium compound having a specific structure that has hydrophilicity and hydrophobicity, and thus, is advantageous in imparting antibacterial properties.

Further, an object of the antibacterial composition according to an exemplary embodiment of the present invention is to secure heat resistance by including a quaternary ammonium compound including an anion other than a halogen as a counterion.

### [Technical Solution]

An exemplary embodiment of the present invention provides an antibacterial composition including a compound represented by the following Chemical Formula 1.

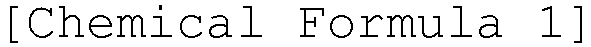

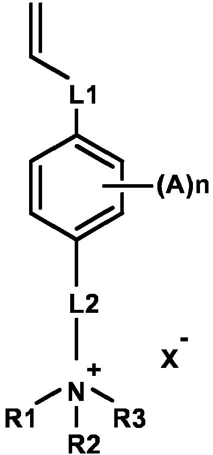

In Chemical Formula 1,
L1 and L2 are the same as or different from each other, and are each independently a direct bond; a substituted or unsubstituted alkylene group having 1 to 4 carbon atoms; or a substituted or unsubstituted arylene group having 6 to 30 carbon atoms,
A is hydrogen; or a substituted or unsubstituted alkyl group having 1 to 3 carbon atoms,
n is an integer from 0 to 4,
two groups of R1 to R3 are the same as or different from each other, and are each independently a substituted or unsubstituted alkyl group having 1 to 4 carbon atoms, and the other groups are a substituted or unsubstituted alkyl group having 3 to 20 carbon atoms,
when n is 2 or higher, two or more A's are the same as or different from each other, and
X⁻ is a hydroxy-based anion, a carbonate-based anion, a citrate-based anion, a cyanate-based anion, a phosphate-based anion, a benzoate-based anion, a sulfonate-based anion, a borate-based anion, a salicylate-based anion, a sulfonamide-based anion, or a sulfonimide-based anion.

### [Advantageous Effects]

The antibacterial composition of the present invention includes a compound having a quaternary ammonium structure and a specific anion, which makes it possible to secure high heat resistance and simultaneously a high level of antibacterial activity.

### [Brief Description of Drawings]

FIG. 1 is a ¹H-NMR spectroscopic spectrum of a compound according to an exemplary embodiment of the present invention.
FIG. 2 is a ¹⁹F-NMR spectroscopic spectrum of a compound according to an exemplary embodiment of the present invention.

### [Best Mode]

Hereinafter, the present invention will be described in detail.

### <Antibacterial composition>

According to an exemplary embodiment of the present invention, provided is an antibacterial composition including a compound represented by the following Chemical Formula 1.

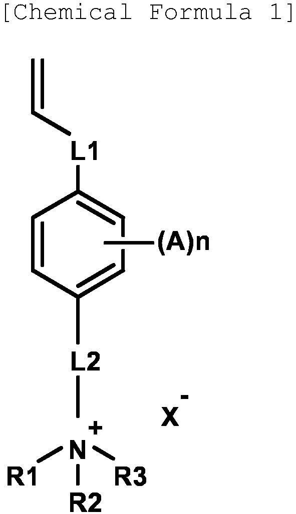

In Chemical Formula 1,
L1 and L2 are the same as or different from each other, and are each independently a direct bond; a substituted or unsubstituted alkylene group having 1 to 4 carbon atoms; or a substituted or unsubstituted arylene group having 6 to 30 carbon atoms,
A is hydrogen; or a substituted or unsubstituted alkyl group having 1 to 3 carbon atoms,
n is an integer from 0 to 4,
two groups of R1 to R3 are the same as or different from each other, and are each independently a substituted or unsubstituted alkyl group having 1 to 4 carbon atoms, and the other group is a substituted or unsubstituted alkyl group having 3 to 20 carbon atoms,
when n is 2 or higher, two or more A's are the same as or different from each other, and
X⁻ is a hydroxy-based anion, a carbonate-based anion, a citrate-based anion, a cyanate-based anion, a phosphate-based anion, a benzoate-based anion, a sulfonate-based anion, a borate-based anion, a salicylate-based anion, a sulfonamide-based anion, or a sulfonimide-based anion.

According to an exemplary embodiment of the present invention, X⁻ may be an anion selected from among trifluoromethanesulfonate, p-toluenesulfonate, tetrafluoroborate, thiocyanate, hexafluorophosphate, salicylate, hydroxybenzoate, carboxyphenolate, trifluoromethylsulfonamidate, trifluoromethanesulfonimidate, bistrifluoromethylsulfonamidate, or bis(trifluoromethyl)sulfonimidate.

According to an exemplary embodiment of the present invention, L1 and L2 are the same as or different from each other, and are each independently a direct bond; or a substituted or unsubstituted alkylene group having 1 to 4 carbon atoms.

According to an exemplary embodiment of the present invention, L1 may be a direct bond; a methylene group or an ethylene group.

According to an exemplary embodiment of the present invention, L1 may be a methylene group.

According to an exemplary embodiment of the present invention, L1 may be a direct bond.

According to an exemplary embodiment of the present invention, L2 may be a direct bond; a methylene group; or an ethylene group.

According to an exemplary embodiment of the present invention, L2 may be a methylene group.

According to an exemplary embodiment of the present invention, L2 may be a direct bond.

According to an exemplary embodiment of the present invention, A may be all hydrogen.

According to an exemplary embodiment of the present invention, two groups of R1 to R3 are the same as or different from each other, and are each independently a methyl group; or an ethyl group, and the other group may be an unsubstituted alkyl group having 8 to 20 carbon atoms.

According to an exemplary embodiment of the present invention, Chemical Formula 1 may be represented by any one of the following structures.

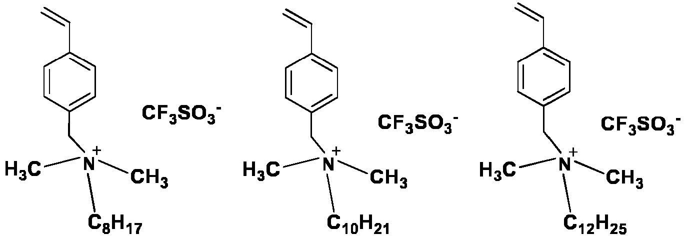

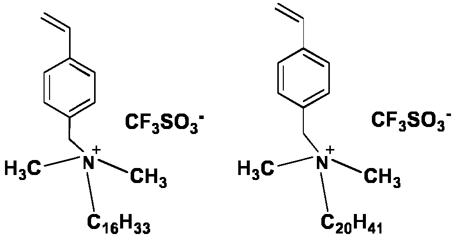

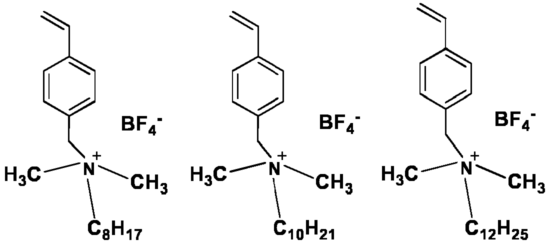

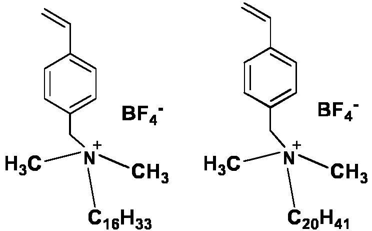

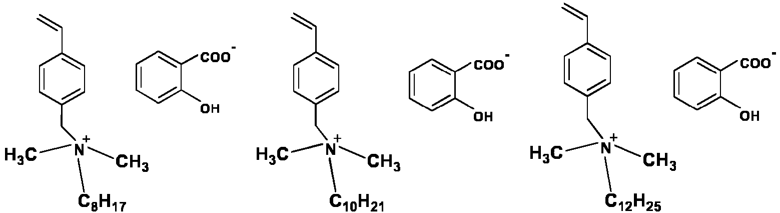

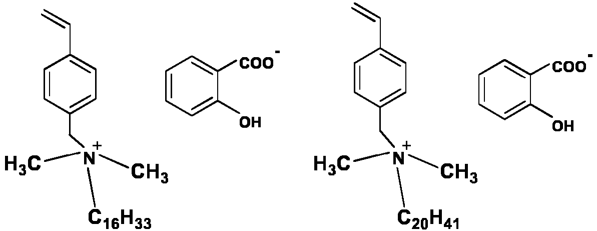

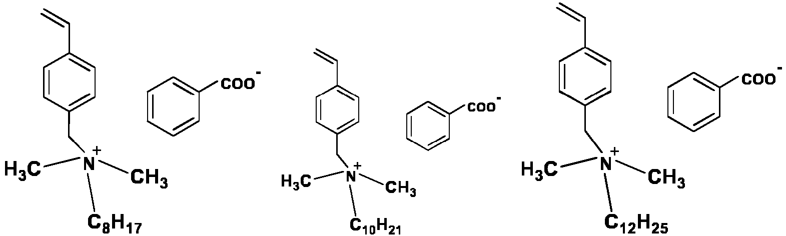

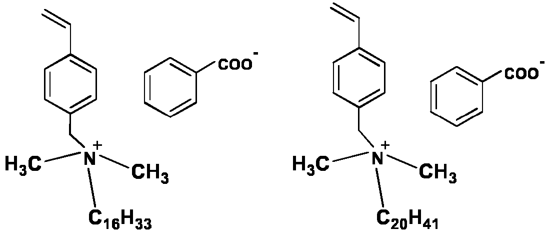

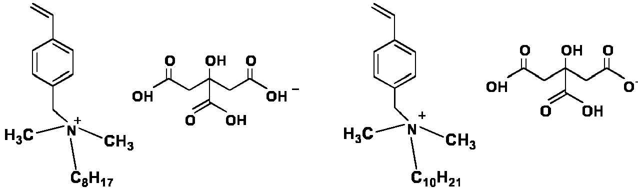

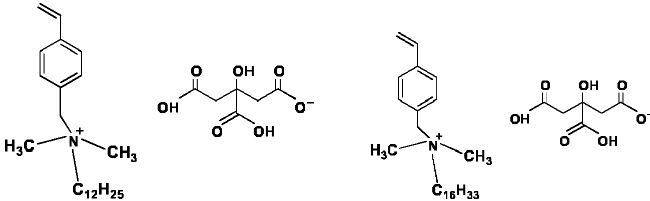

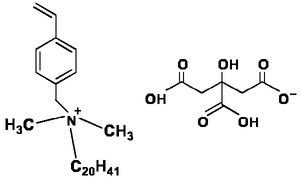

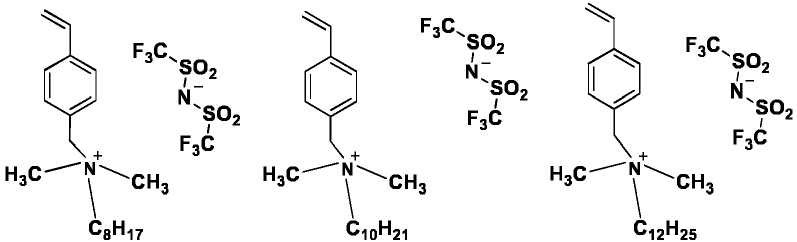

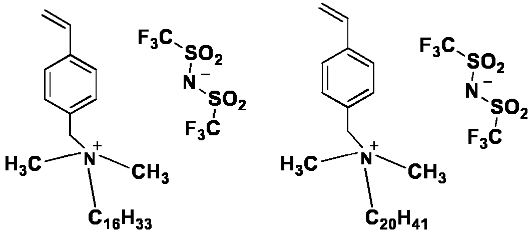

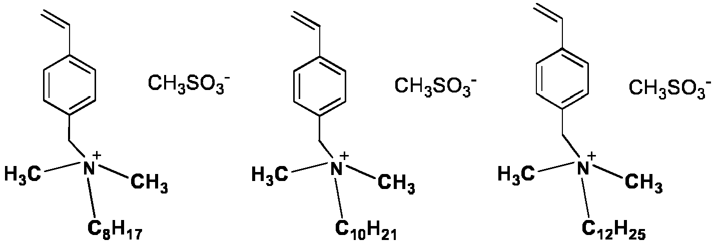

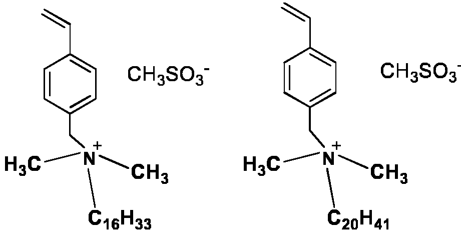

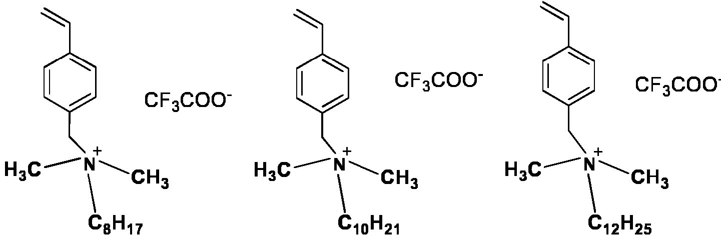

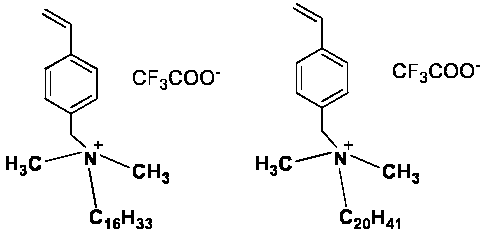

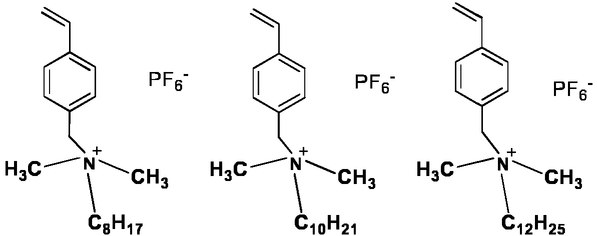

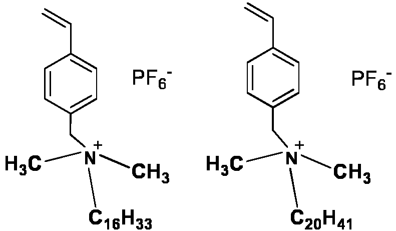

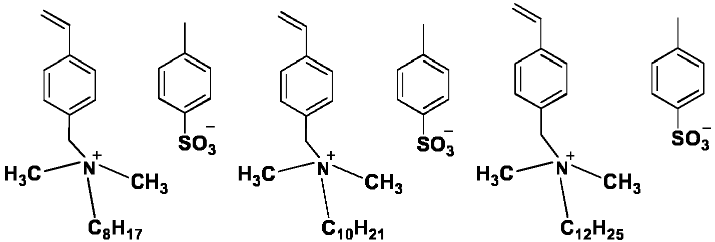

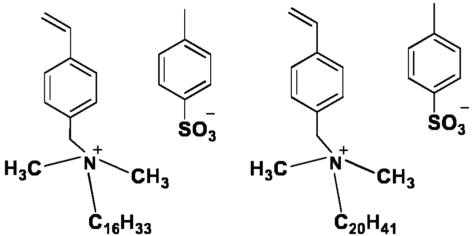

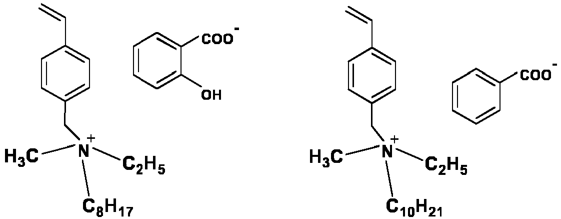

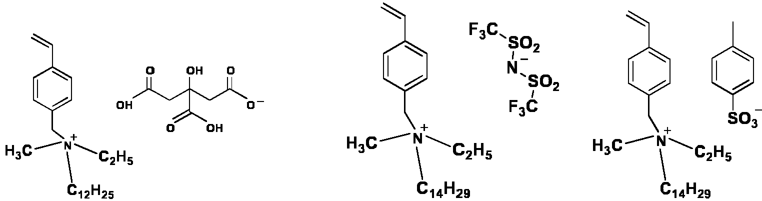

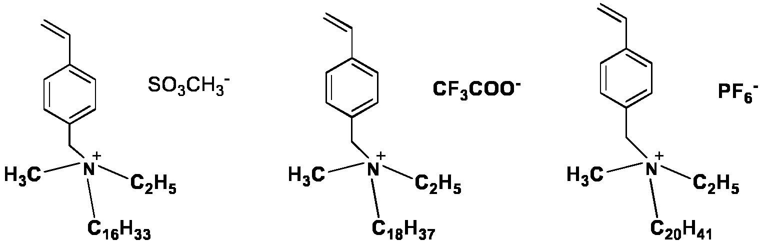

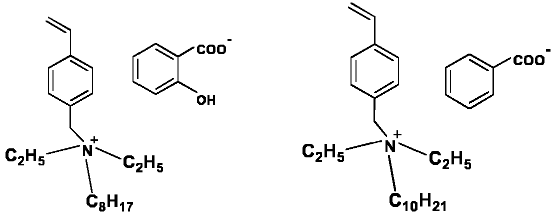

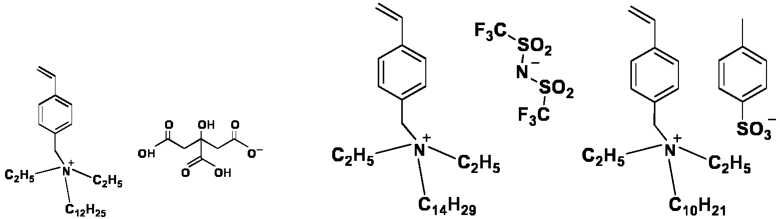

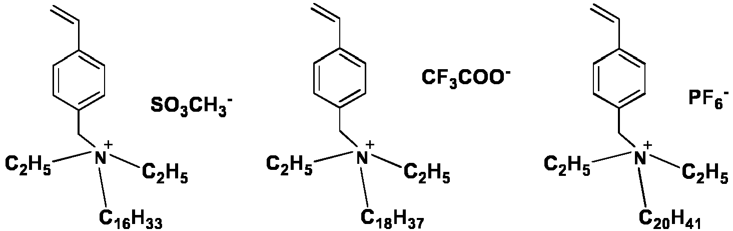

According to an exemplary embodiment of the present invention, the primary thermal decomposition temperature of the antibacterial composition may be 200°C or higher.

In the present invention, the thermal decomposition temperature may be measured using a thermogravimetric analyzer.

The primary thermal decomposition temperature may be defined as an extrapolated intersection point between a starting mass reference line and the tangent line of the maximum slope point in the first mass reduction section of a mass loss curve measured by a thermogravimetric analyzer in an N₂ atmospheric environment.

In the present invention, it is determined that when the primary thermal decomposition temperature of the antibacterial composition has a value higher than the primary thermal decomposition temperature of the material before substitution, the heat resistance has increased. However, in consideration of the processing and application processes of a polymer using the compound, it is determined that the polymer has thermal stability only when the primary thermal decomposition temperature is 200°C or higher.

As long as the primary thermal decomposition temperature of the antibacterial composition is 200°C or higher, the upper limit thereof is not particularly limited, and it means that the higher the primary thermal decomposition temperature, the better the heat resistance.

In the present specification, the primary thermal decomposition temperature is the temperature at which thermal decomposition occurs in the quaternary ammonium group of the compound, and the present inventors found that when the compound is substituted with a specific anion other than a halogen anion as an anion group (that is, X⁻), the thermal stability of the quaternary ammonium group increases, and as a result, the thermal decomposition temperature increases. Meanwhile, in secondary and higher thermal decomposition, thermal decomposition occurs in areas other than the quaternary ammonium group, so that the primary thermal decomposition experiment is used as the reference, as it is easier to compare effects under the same conditions.

Furthermore, as described above, when the thermal decomposition temperature of the quaternary ammonium compound (that is, monomer) substituted with the specific anion increases, a polymer including the compound provides an effect in which resistance is high in processes in a high temperature environment.

According to an exemplary embodiment of the present invention, the antibacterial composition may have a bacterial proliferation inhibition rate of 70% or more against at least one strain of Gram-positive bacteria, Gram-negative bacteria, and fungi, as measured by the following Method 1.

### [Method 1]

After 0.04 g of the antibacterial composition is added to 20 mL of a nutrient broth culture solution inoculated with 3,000 ± 300 CFU/mL of bacterial strain, from the culture solution of an experimental group in which the resulting mixture is incubated at 37°C in a shaking incubator for 24 hours, the absorbance at a wavelength of 600 nm is measured using a UV/Vis spectrophotometer,
the antibacterial composition is not added to 20 mL of a nutrient broth culture solution inoculated with 3,000 ± 300 CFU/mL of bacterial strain, from the culture solution of a control in which the nutrient broth culture solution is incubated at 37°C for 24 hours, the absorbance at a wavelength of 600 nm is measured using a UV/Vis spectrophotometer, and
from the absorbance of the experimental group and the absorbance of the control group, the bacterial proliferation inhibition rate (%) of the test bacteria is calculated using the following Equation 1. Bacterial proliferation inhibition rate= (1- AExperimental group/AControl) × 100%
   A_{Experimental group} = Absorbance of culture solution of experimental group
   A_{Control} = Absorbance of culture solution of control

According to an exemplary embodiment of the present invention, the bacterial proliferation inhibition rate of the compound according to Method 1 may be 50% or more, 60% or more, 70% or more, 80% or more, or 90% or more.

In the present invention, "having antibacterial properties" means that the bacterial proliferation inhibition rate (%) is 50% or more, 60% or more, preferably 70% or more, more preferably 80% or more.

In the present invention, "having antibacterial properties" means that the bacterial proliferation inhibition rate (%) according to Method 1 is 70% or more, preferably 80% or more.

According to an exemplary embodiment of the present invention, the Gram-positive bacterium may be any one selected from among *Enterococcus faecalis, Staphylococcus aureus, Streptococcus pneumoniae, Streptococcus pyogene, Enterococcus faecium,* and *Lactobacillus lactis,* but is not limited thereto.

As used herein, the term Gram-positive bacteria is a general term for bacteria that are stained purple when stained using the Gram staining method, and Gram-positive bacteria exhibit a purple color without discoloration even though the Gram-positive bacteria are stained with a basic dye such as crystal violet and then treated with ethanol because the cell walls of Gram-positive bacteria are composed of several layers of peptidoglycan.

The Gram-negative bacterium may be any one selected from among *Proteus mirabilis, Escherichia coli, Salmonella typhi, Pseudomonas aeruginosa, Vibrio cholerae,* and *Enterobacter cloacae* as a bacterium, but is not limited thereto.

As used herein, the term Gram-negative bacteria is a general term for bacteria that are stained red when stained using the Gram staining method, and Gram-negative bacteria have an outer membrane composed of lipid polysaccharides, lipid proteins, and/or other complex polymeric materials instead of having a cell wall with a relatively small amount of peptidoglycan compared to Gram-positive bacteria.

According to an exemplary embodiment of the present invention, the fungus may be *Candida albicans,* and the like, but is not limited thereto.

Since the bacterial strains of the Gram-positive bacteria, Gram-negative bacteria and fungi may not only induce various diseases upon contact, but also cause secondary infections, it is preferred to exhibit antibacterial properties against all of the Gram-positive bacteria, Gram-negative bacteria, and fungi using one antibacterial compound.

When one member (layer) is disposed "on" another member (layer) in the present invention, this includes not only a case where the one member (layer) is brought into contact with another member, but also a case where still another member (layer) is present between the two members (layers).

When one part "includes" one constituent element in the present invention, unless otherwise specifically described, this does not mean that another constituent element is excluded, but means that another constituent element may be further included.

In the present invention, the "monomer" means a unit compound that may be converted into a polymer compound by a polymerization reaction, that is, a monomer, and structures derived therefrom may become a repeating unit in a polymer or copolymer. Specifically, this means that in a state in which the corresponding compound is polymerized and bonded in the polymer, in the structure of the compound, all or a portion of two or more substituents are omitted, and a radical for being bonded to other units of the polymer is located at the position. In this case, the corresponding compound may be included in a state of being polymerized in any order and bonded in the polymer.

### [Mode for Invention]

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present invention are provided for more completely explaining the present invention to the person with ordinary skill in the art.

### Synthesis Examples: Synthesis of compounds.

### <Synthesis Example 1>

The reaction scheme of the present invention is as follows.

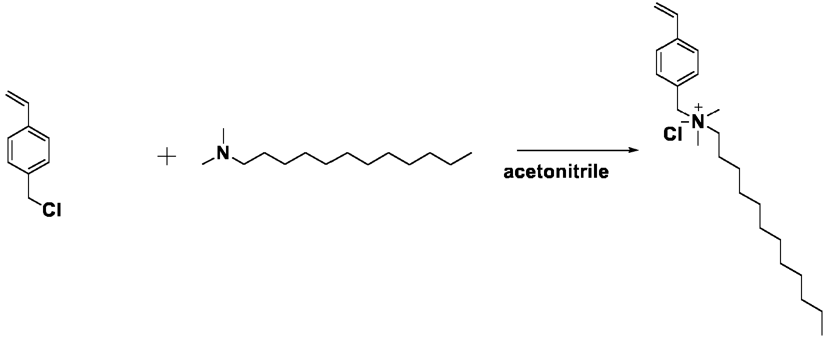

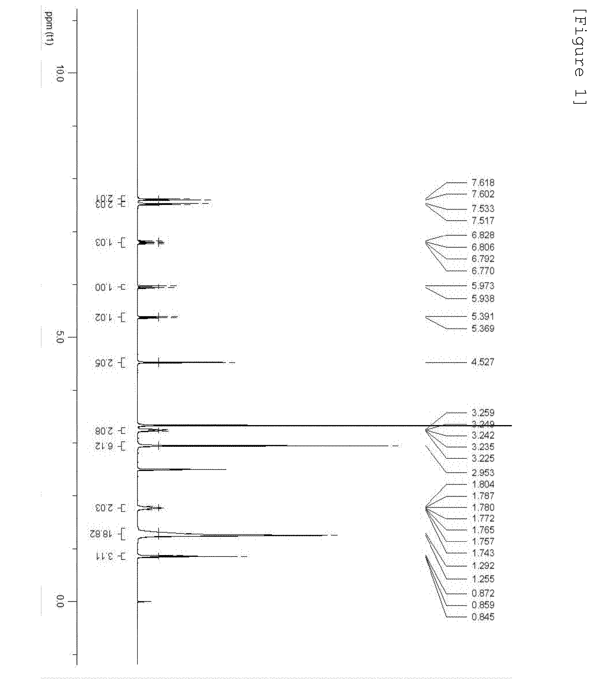

A compound having a halogen anion (or an antibacterial monomer) used in the present invention was prepared as described above. A reactor in the form of a two-neck round flask connected to a blade-type stirrer to which a condenser and a stirrer are connected was prepared. 50 g of 1-chloromethyl-4-vinyl benzene and 81.3 g of N,N-dimethyldodecyl amine were mixed with acetonitrile (150 mL) and then put into the reactor. After the inside of the reactor was substituted with a nitrogen atmosphere, the temperature was increased to 45°C, and a reaction was performed with stirring for 24 hours or more. A solution in which the reaction had been completed was added to hexane to precipitate and purify the reaction product. After the purification process was additionally performed two or more times, Compound 1 was obtained by drying the purified reaction product at about 80°C for 24 hours or more. See FIG. 1, for the ¹H-NMR spectrum of Compound 1.

### <Synthesis Example 2>

Compound 2 was prepared in the same manner as in Synthesis Example 1, except that 81.3 g of N,N-dimethyldodecyl amine was changed to 56.7 g of N,N-dimethyloctyl amine. It was confirmed by ¹H-NMR spectrum that Compound 2 was synthesized in a manner similar to Compound 1.

### <Synthesis Example 3>

Compound 3 was prepared in the same manner as in Synthesis Example 1, except that 81.3 g of N,N-dimethyldodecyl amine was changed to 69.0 g of N,N-dimethyldecyl amine. It was confirmed by ¹H-NMR spectrum that Compound 3 was synthesized in a manner similar to Compound 1.

### <Synthesis Example 4>

Compound 4 was prepared in the same manner as in Synthesis Example 1, except that 81.3 g of N,N-dimethyldodecyl amine was changed to 105.9 g of N,N-dimethylcetyl amine. It was confirmed by ¹H-NMR spectrum that Compound 4 was synthesized in a manner similar to Compound 1.

### <Synthesis Example 5>

Compound 5 was prepared in the same manner as in Synthesis Example 1, except that 81.3 g of N,N-dimethyldodecyl amine was changed to 130.6 g of N,N-dimethyl eicosanamine. It was confirmed by ¹H-NMR spectrum that Compound 5 was synthesized in a manner similar to Compound 1.

### Examples: Anion substitution (preparation of antibacterial composition)

### <Example 1>

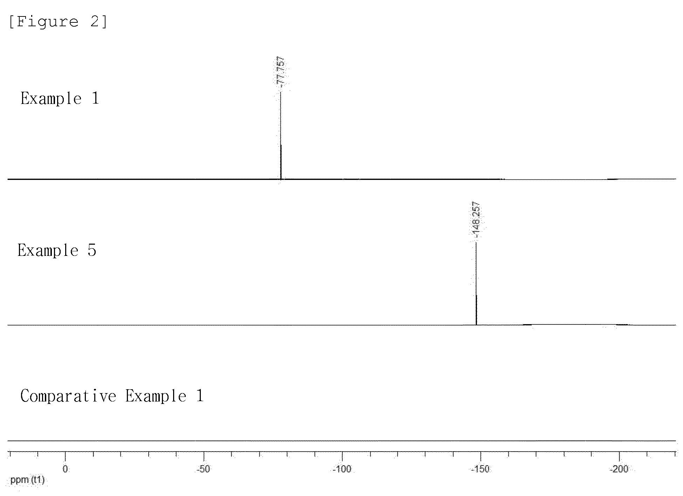

1 g of Compound 1 prepared in Synthesis Example 1 was dissolved in 10 g of water. After 0.94 g of sodium trifluoromethyl sulfonate was dissolved in 10 g of water in another container, the resulting solution was added to the reactor and stirred for 8 hours or more to substitute the anion with a trifluoromethyl sulfonate anion (CF₃SO₃⁻) from a halogen anion (that is, Cl⁻). An excess amount of ethyl acetate was added to and mixed with the substitution solution, and the mixed solution was allowed to stand for 30 minutes or more to separate phases. After only the upper part of ethyl acetate was separated from the separated mixed solution, MgSO₄ was added thereto to remove the remaining water. After MgSO₄ in the ethyl acetate solution was filtered, the ethyl acetate was removed from a 40°C vacuum oven to obtain the antibacterial composition of Example 1. According to FIG. 2, it was confirmed in the ¹⁹F-Nuclear Magnetic Resonance (NMR, Ascend^{™} 500 manufactured by Bruker) spectroscopic results that the F element of trifluoromethyl sulfonate was detected, so that it was confirmed that the anion of Compound 1 of Synthesis Example 1 was substituted with trifluoromethyl sulfonate containing a F element from Cl⁻.

### <Example 2>

The antibacterial composition of Example 2 was obtained by performing the same method as in Example 1, except that 1 g of Compound 1 was changed to 1 g of Compound 2 prepared in Synthesis Example 2. It was confirmed by ¹⁹F-NMR spectrum that the antibacterial composition of Example 2 was subjected to anion substitution in a manner similar to the compound of Example 1.

### <Example 3>

The antibacterial composition of Example 3 was obtained by performing the same method as in Example 1, except that 1 g of Compound 1 was changed to 1 g of Compound 3 prepared in Synthesis Example 3. It was confirmed by ¹⁹F-NMR spectrum that the antibacterial composition of Example 3 was subjected to anion substitution in a manner similar to the compound of Example 1.

### <Example 4>

The antibacterial composition of Example 4 was obtained by performing the same method as in Example 1, except that 1 g of Compound 1 was changed to 1 g of Compound 4 prepared in Synthesis Example 4. It was confirmed by ¹⁹F-NMR spectrum that the antibacterial composition of Example 4 was subjected to anion substitution in a manner similar to the compound of Example 1.

### <Example 5>

The antibacterial composition of Example 5 was obtained by performing the same method as in Example 1, except that 1 g of sodium trifluoromethyl sulfonate was changed to 0.60 g of sodium tetrafluoro borate. According to FIG. 2, it was confirmed in the ¹⁹F-NMR spectroscopic results that the F element of tetrafluoro borate was detected, so that it was confirmed that the anion of Compound 1 of Synthesis Example 1 was substituted with tetrafluoro borate containing a F element from Cl⁻.

### <Example 6>

The antibacterial composition of Example 6 was obtained by performing the same method as in Example 1, except that 1 g of sodium trifluoromethyl sulfonate was changed to 0.87 g of sodium salicylate. It was indirectly confirmed by the ¹⁹F-NMR spectrum of the compound of Example 1 that the antibacterial composition of Example 6 was substituted with F⁻ from Cl⁻, and it was confirmed that anions were substituted with salicylate from F⁻ by confirming that an F peak disappeared during substitution with salicylate.

### <Example 7>

The antibacterial composition of Example 7 was obtained by performing the same method as in Example 1, except that 1 g of sodium trifluoromethyl sulfonate was changed to 1.65 g of sodium bis(trifluoro methane) sulfonamide. It was confirmed by ¹⁹F-NMR spectrum that the antibacterial composition of Example 7 was subjected to anion substitution in a manner similar to the compound of Example 1.

### <Example 8>

The antibacterial composition of Example 8 was obtained by performing the same method as in Example 1, except that 1 g of sodium trifluoromethyl sulfonate was changed to 0.65 g of sodium methane sulfonate. It was confirmed by the ¹⁹F-NMR spectrum of Example 1 that the antibacterial composition of Example 8 was substituted with F⁻ from Cl⁻, and it was indirectly confirmed that anions were substituted with methane sulfonate from F⁻ by confirming that an F peak disappeared during substitution with methane sulfonate.

### <Example 9>

The antibacterial composition of Example 9 was obtained by performing the same method as in Example 1, except that 1 g of sodium trifluoromethyl sulfonate was changed to 0.79 g of sodium benzoate. It was confirmed by the ¹⁹F-NMR spectrum of Example 1 that the antibacterial composition of Example 9 was substituted with F⁻ from Cl⁻, and it was indirectly confirmed that anions were substituted with benzoate from F⁻ by confirming that an F peak disappeared during substitution with benzoate.

### <Example 10>

The antibacterial composition of Example 10 was obtained by performing the same method as in Example 1, except that 1 g of sodium trifluoromethyl sulfonate was changed to 1.41 g of sodium citrate monobasic. It was confirmed by the ¹⁹F-NMR spectrum of Example 1 that the antibacterial composition of Example 10 was substituted with F⁻ from Cl⁻, and it was indirectly confirmed that anions were substituted with citrate monobasic from F⁻ by confirming that an F peak disappeared during substitution with citrate monobasic.

### <Example 11>

The antibacterial composition of Example 11 was obtained by performing the same method as in Example 1, except that 1 g of sodium trifluoromethyl sulfonate was changed to 0.74 g of sodium trifluoroacetate. It was confirmed by ¹⁹F-NMR spectrum that the antibacterial composition of Example 11 was subjected to anion substitution in a manner similar to the compound of Example 1.

### <Example 12>

The antibacterial composition of Example 12 was obtained by performing the same method as in Example 1, except that 1 g of sodium trifluoromethyl sulfonate was changed to 1.01 g of potassium hexafluorophosphate. It was confirmed by ¹⁹F-NMR spectrum that the antibacterial composition of Example 12 was subjected to anion substitution in a manner similar to the compound of Example 1.

### <Example 13>

The antibacterial composition of Example 13 was obtained by performing the same method as in Example 1, except that 1 g of sodium trifluoromethyl sulfonate was changed to 0.53 g of potassium thiocyanate. It was confirmed by the ¹⁹F-NMR spectrum of Example 1 that the antibacterial composition of Example 13 was substituted with F⁻ from Cl⁻, and it was indirectly confirmed that anions were substituted with thiocyanate from F⁻ by confirming that an F peak disappeared during substitution with thiocyanate.

### <Example 14>

The antibacterial composition of Example 14 was obtained by performing the same method as in Example 1, except that 1 g of sodium trifluoromethyl sulfonate was changed to 1.06 g of sodium p-toluenesulfonate. It was confirmed by the ¹⁹F-NMR spectrum of Example 1 that the antibacterial composition of Example 14 was substituted with F⁻ from Cl⁻, and it was indirectly confirmed that anions were substituted with p-toluenesulfonate from F⁻ by confirming that an F peak disappeared during substitution with p-toluenesulfonate.

### <Comparative Example 1>

As a compound not subjected to anion substitution as in the Examples, Compound 1 of Synthesis Example 1 was selected as the compound of Comparative Example 1. According to FIG. 2, it was confirmed in the ¹⁹F-NMR spectroscopic results that the F element was not detected, so that it can be assumed that the anion (Cl⁻) of Compound 1 of Synthesis Example 1 remains as it is.

### Experimental Examples.

### <Experimental Example 1: Primary thermal decomposition measurement experiment>

A thermogravimetric analyzer (TGA2 manufactured by Mettler Toledo) was used in order to perform an experiment on the primary thermal decomposition temperature of the antibacterial compositions of Examples 1 to 13 and Comparative Example 1.

In the present experiment, the primary thermal decomposition temperature may be defined as an extrapolated intersection point between a starting mass reference line and the tangent line of the maximum slope point in the first mass reduction section of a mass loss curve measured by a thermogravimetric analyzer in an N₂ atmospheric environment. In the present invention, it was determined that when the primary thermal decomposition temperature of the antibacterial composition has a value higher than the primary thermal decomposition temperature of the material before anion substitution, the heat resistance has increased. The measurement results are shown in the following Table 1.

### <Experimental Example 2: Bacterial proliferation inhibition rate (%) measurement experiment>

In the present experiment, the bacterial proliferation inhibition rate was measured according to the following Method 1. In this case, *E.coli* bacteria were used. The measurement results are shown in the following Table 1.

### [Method 1]

After 0.04 g of the antibacterial composition is added to 20 mL of a nutrient broth culture solution inoculated with 3,000 ± 300 CFU/mL of bacterial strain, from the culture solution of an experimental group in which the resulting mixture is incubated at 37°C in a shaking incubator for 24 hours, the absorbance at a wavelength of 600 nm is measured using a UV/Vis spectrophotometer,
the antibacterial composition is not added to 20 mL of a nutrient broth culture solution inoculated with 3,000 ± 300 CFU/mL of bacterial strain, from the culture solution of a control in which the nutrient broth culture solution is incubated at 37°C for 24 hours, the absorbance at a wavelength of 600 nm is measured using a UV/Vis spectrophotometer, and
from the absorbance of the experimental group and the absorbance of the control group, the bacterial proliferation inhibition rate (%) of the test bacteria is calculated using the following Equation 1. Bacterial proliferation inhibition rate= (1- AExperimental group/AControl) × 100%
   A_{Experimental group} = Absorbance of culture solution of experimental group
   A_{Control} = Absorbance of culture solution of control

**[Table 1]**

| No. | Primary thermal decomposition temperature1 (°C) | Bacterial proliferation inhibition rate (%) |
|---|---|---|
| Example 1 | 369 | 93.3 |
| Example 2 | 353 | 91.4 |
| Example 3 | 362 | 93.1 |
| Example 4 | 359 | 95.7 |
| Example 5 | 335 | 98.6 |
| Example 6 | 208 | 93.5 |
| Example 7 | 384 | 95.8 |
| Example 8 | 205 | 93.2 |
| Example 9 | 204 | 96.4 |
| Example 10 | 202 | 94.2 |
| Example 11 | 201 | 95.4 |
| Example 12 | 309 | 93.2 |
| Example 13 | 211 | 94.7 |
| Example 14 | 233 | 95.2 |
| Comparative Example 1 | 176 | 96.3 |

According to those described in Table 1 above, it was found that Examples 1 to 14 are antibacterial compositions substituted with anions other than halogens, exhibit a bacterial proliferation inhibition rate of 90% or more to maintain high antibacterial properties, and simultaneously, the primary thermal decomposition temperature exceeded 200°C, indicating high thermal resistance. In contrast, it was found that Comparative Example 1 including halogen anions has a primary thermal decomposition temperature of 200°C or less, and thus, has low thermal resistance even though a high level of antibacterial properties was maintained.

## Claims

1. An antibacterial composition comprising a compound represented by the following Chemical Formula 1: 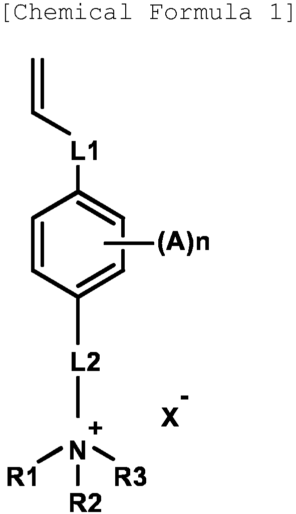 wherein, in Chemical Formula 1,
L1 and L2 are the same as or different from each other, and are each independently a direct bond; a substituted or unsubstituted alkylene group having 1 to 4 carbon atoms; or a substituted or unsubstituted arylene group having 6 to 30 carbon atoms,
A is hydrogen; or a substituted or unsubstituted alkyl group having 1 to 3 carbon atoms,
n is an integer from 0 to 4,
two groups of R1 to R3 are the same as or different from each other, and are each independently a substituted or unsubstituted alkyl group having 1 to 4 carbon atoms, and the other group is a substituted or unsubstituted alkyl group having 3 to 20 carbon atoms,
when n is 2 or higher, two or more A's are the same as or different from each other, and
X⁻ is a hydroxy-based anion, a carbonate-based anion, a citrate-based anion, a cyanate-based anion, a phosphate-based anion, a benzoate-based anion, a sulfonate-based anion, a borate-based anion, a salicylate-based anion, a sulfonamide-based anion, or a sulfonimide-based anion.

2. The antibacterial composition of claim 1, wherein X-is an anion selected from among trifluoromethanesulfonate, p-toluenesulfonate, tetrafluoroborate, thiocyanate, hexafluorophosphate, salicylate, hydroxybenzoate, carboxyphenolate, trifluoromethylsulfonamidate, trifluoromethanesulfonimidate, bistrifluoromethylsulfonamidate, or bis(trifluoromethyl)sulfonimidate.

3. The antibacterial composition of claim 1, wherein L1 is a direct bond.

4. The antibacterial composition of claim 1, wherein L2 is a methylene group.

5. The antibacterial composition of claim 1, wherein A is hydrogen.

6. The antibacterial composition of claim 1, wherein two groups of R1 to R3 are the same as or different from each other, and are each independently a methyl group; or an ethyl group, and the other group is an unsubstituted alkyl group having 8 to 20 carbon atoms.

7. The antibacterial composition of claim 1, wherein Chemical Formula 1 is represented by any one of the following structures:

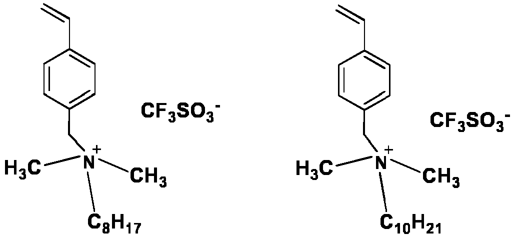

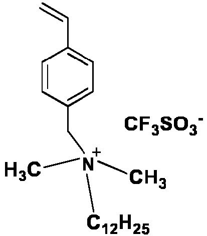

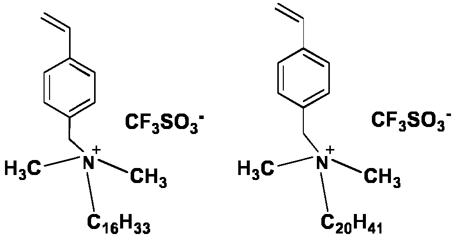

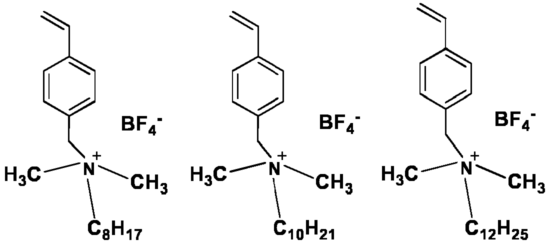

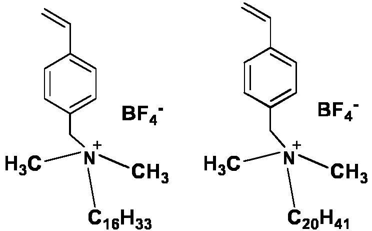

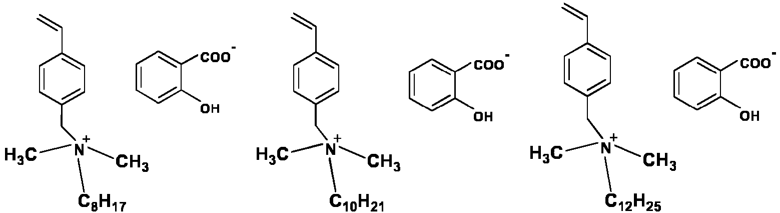

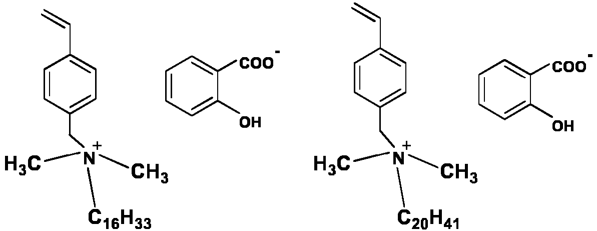

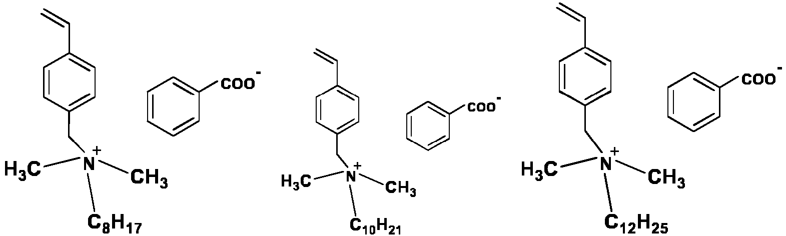

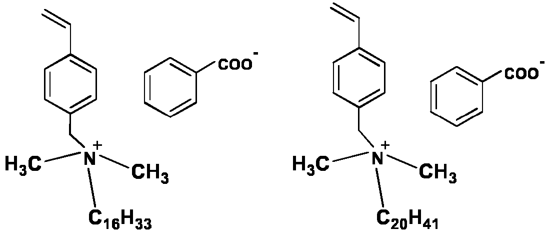

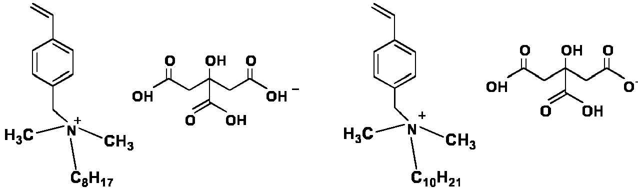

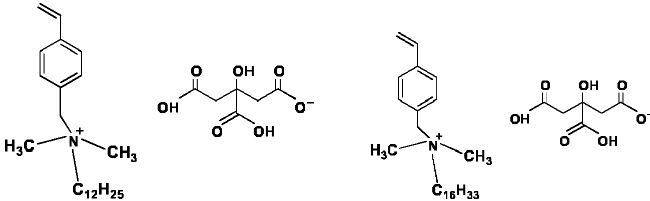

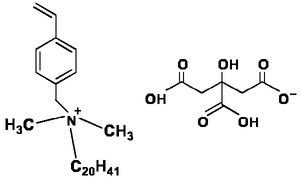

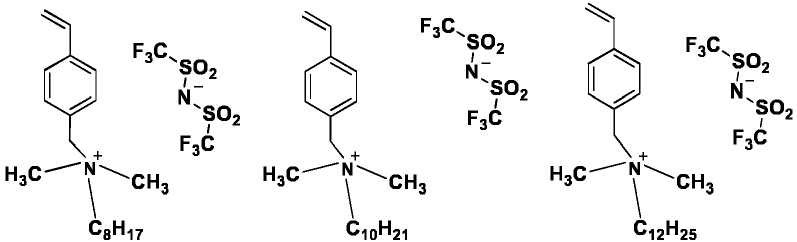

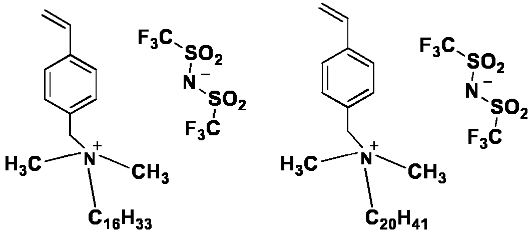

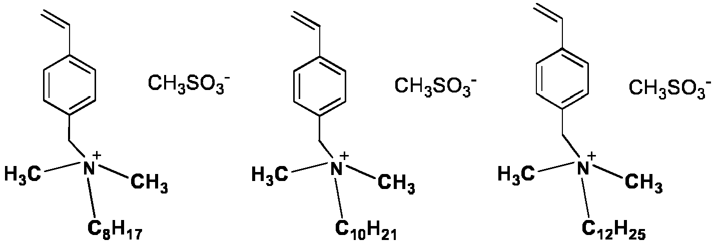

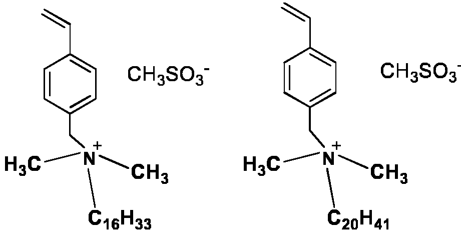

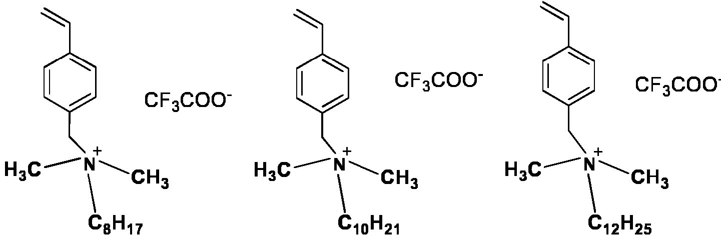

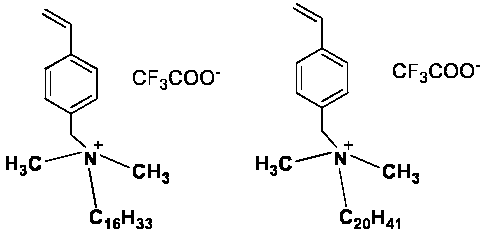

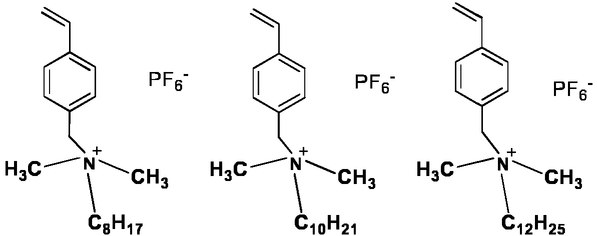

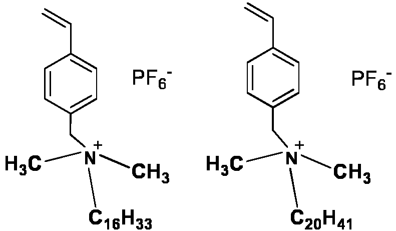

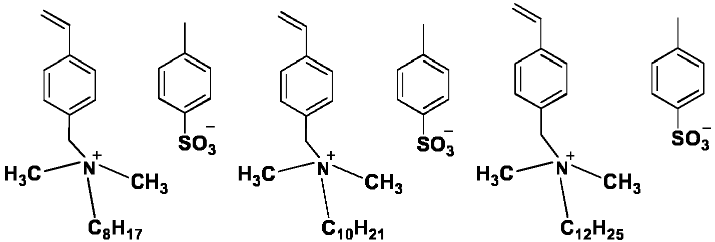

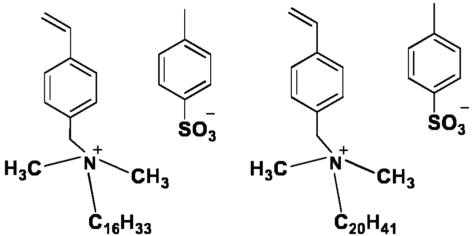

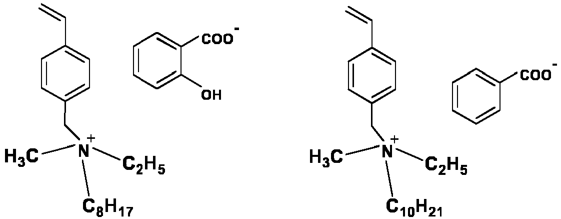

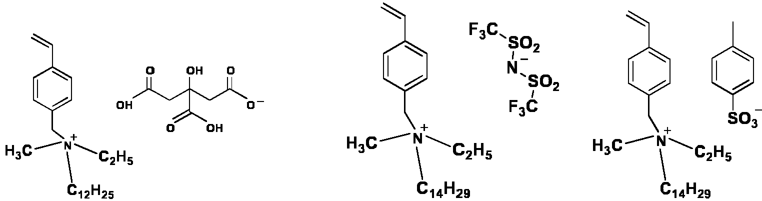

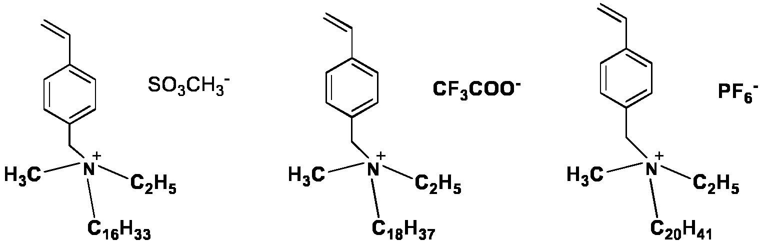

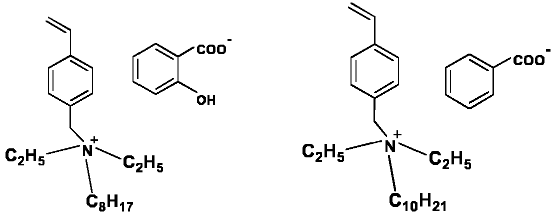

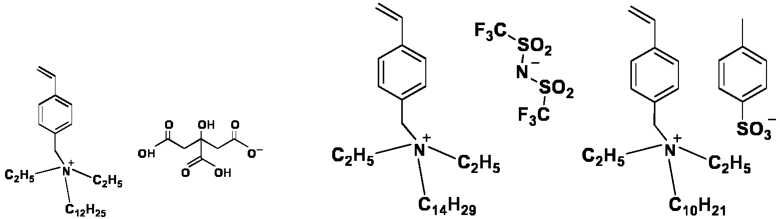

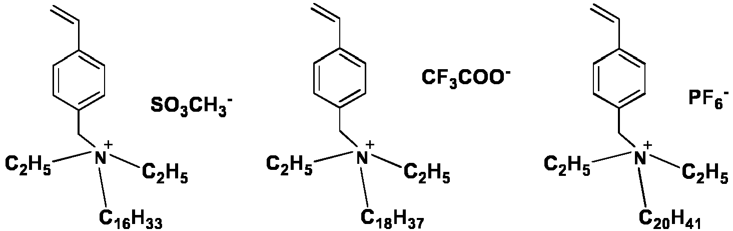

8. The antibacterial composition of claim 1, wherein the primary thermal decomposition temperature of the compound is 200°C or higher.

9. The antibacterial composition of claim 1, wherein the compound has a bacterial proliferation inhibition rate of 70% or more against at least one strain of Gram-positive bacteria, Gram-negative bacteria, and fungi, as measured by the following Method 1:
[Method 1]
After 0.04 g of the antibacterial composition is added to 20 mL of a nutrient broth culture solution inoculated with 3,000 ± 300 CFU/mL of bacterial strain, from the culture solution of an experimental group in which the resulting mixture is incubated at 37°C in a shaking incubator for 24 hours, the absorbance at a wavelength of 600 nm is measured using a UV/Vis spectrophotometer,
the antibacterial composition is not added to 20 mL of a nutrient broth culture solution inoculated with 3,000 ± 300 CFU/mL of bacterial strain, from the culture solution of a control in which the nutrient broth culture solution is incubated at 37°C for 24 hours, the absorbance at a wavelength of 600 nm is measured using a UV/Vis spectrophotometer, and
from the absorbance of the experimental group and the absorbance of the control group, the bacterial proliferation inhibition rate (%) of the test bacteria is calculated using the following Equation 1. Bacterial proliferation inhibition rate= (1- AExperimental group/AControl) × 100%
A_{Experimental group} = Absorbance of culture solution of experimental group
A_{Control} = Absorbance of culture solution of control

10. The antibacterial composition of claim 9, wherein the Gram-positive bacterium is any one selected from among *Enterococcus faecalis, Staphylococcus aureus, Streptococcus pneumoniae, Streptococcus pyogene, Enterococcus faecium,* and *Lactobacillus lactis.*

11. The antibacterial composition of claim 9, wherein the Gram-negative bacterium is any one selected from among *Proteus mirabilis, Escherichia coli, Salmonella typhi, Pseudomonas aeruginosa, Vibrio cholerae,* and *Enterobacter cloacae* as a bacterium.
